# EUROPÄISCHE PATENTSCHRIFT

(11) **EP 2 480 150 B1**
(45) Veröffentlichungstag und Bekanntmachung des Hinweises auf die Patenterteilung: **27.03.2019**
(21) Anmeldenummer: 10757048.3
(22) Anmeldetag: 21.09.2010
(51) Int. Cl.: A61B 18/02, A61B 18/00, A61B 18/12

(54) **VERSORGUNGSEINRICHTUNG ZUM BETREIBEN MINDESTENS EINES MEDIZINISCHEN INSTRUMENTS**
SUPPLY DEVICE FOR OPERATING AT LEAST ONE MEDICAL INSTRUMENT
DISPOSITIF D'ALIMENTATION POUR FAIRE FONCTIONNER AU MOINS UN INSTRUMENT MÉDICAL

(30) Priorität: 21.09.2009 DE 102009042428
(43) Veröffentlichungstag der Anmeldung: 01.08.2012
(73) Patentinhaber: Erbe Elektromedizin GmbH, 72072 Tübingen (DE)
(72) Erfinder: FRITZ, Martin, 72070 Tübingen (DE); SCHALL, Heiko, 72622 Nürtingen (DE)
(74) Vertreter: Rüger Abel
(86) Internationale Anmeldenummer: PCT/EP2010/005776
(87) Internationale Veröffentlichungsnummer: WO 2011/032729

(56) Entgegenhaltungen:
- WO-A1-96/13216
- WO-A1-03/090849
- US-A- 5 309 919
- US-A1- 2003 046 658
- US-A1- 2003 237 027
- US-A1- 2004 054 297
- US-A1- 2007 179 495

## Beschreibung

Die Erfindung betrifft eine Versorgungseinrichtung zum Betreiben mindestens eines medizinischen Instruments gemäß dem Anspruch 1.

In den letzten Jahren gewinnt die Steuer- und Regelungstechnik auch für die Medizin zunehmende Bedeutung. So werden bei zahlreichen Operationen Geräte verwendet, die eine Vielzahl von elektronischen Bauteilen umfassen. Üblicherweise setzen sich diese medizinischen Geräte aus mindestens einem Instrument und einer Versorgungseinrichtung zusammen, die das Instrument entsprechend versorgt. Beispielsweise sind elektronisch gesteuerte kryochirurgische, elektrochirurgische und wasserstrahlchirurgische Instrumente bekannt, die mittels sehr komplexer Steuer- und Regelalgorithmen betrieben werden. So wird beispielsweise bei der Hochfrequenzchirurgie (HF- Chirurgie) Wechselstrom mit hoher Frequenz durch den menschlichen Körper geleitet, um Gewebe gezielt zu devitalisieren bzw. zu schneiden. In der Hochfrequenzchirurgie wird ein HF-Generator an ein entsprechendes elektrochirurgisches Instrument angeschlossen, um einen geeigneten Wechselstrom bereitzustellen. In der Kryochirurgie werden Versorgungseinrichtungen verwendet, die in fluider Verbindung mit entsprechenden Instrumenten (beispielsweise Kryosonden) stehen und ein Fluid zur Kühlung des Instruments bereitstellen. Es ist offensichtlich, dass die entsprechenden Versorgungseinrichtungen über Steuereinheiten, Eingabeeinheiten und Ausgabeeinheiten verfügen müssen, um die angeschlossenen Instrumente geeignet zu steuern.

Entsprechende elektrochirurgische Geräte sind aus der EP 1 862 137 A1 und EP 1 829 493 A1 bekannt. Beim Steuern dieser Geräte müssen zahlreiche Parameter (beispielsweise Gewebeart, Betriebsmodus, usw.) berücksichtigt werden. Die in den Versorgungseinrichtungen enthaltenen Steuereinheiten regeln und steuern Stellgrößen (wie beispielsweise die Spannung, den Strom, die Leistung, die Modulationsfrequenz usw.). Häufig bieten elektrochirurgische Versorgungseinrichtungen mehrere Betriebsmodi, beispielsweise einen Modus zum Schneiden und einen zum Koagulieren, an. In der Vergangenheit wurde für jeden dieser Betriebsmodi ein eigenes Regel- bzw. Steuerprogramm entwickelt. Die Pflege dieser Programme gestaltet sich als äußerst aufwendig und fehleranfällig. Des Weiteren lässt sich die Korrektheit von entsprechenden Steuerprogrammen nicht deterministisch feststellen, so dass zahlreiche Tests notwendig sind, um die Sicherheit des zu behandelnden Patienten und des Personals zu gewährleisten. Die Entwicklung der Regel- und Steuerprogramme ist somit sehr teuer.

Aus der US 2007/0179495 A1 ist eine Versorgungseinrichtung zum Betreiben eines medizinischen Instruments bekannt, das eine Steuereinheit mit Bedienelementen zur Steuerung der Leistungsabgabe des Generators aufweist. Über die Bedienelemente können Voreinstellungen, beispielsweise hinsichtlich der Auswahl von Elektrodenflächen oder hinsichtlich der Einstellung des Generators vorgenommen und abgespeichert werden, so dass der Chirurg Einstellungen des Generators bedarfsweise abrufen kann und nicht jedes Mal neu einstellen muss. Die Steuereinheit kann dann den Generator anhand der abgespeicherten Einstellungen steuern.

Aus der US 2003/0237027 A1 ist eine Programmumgebung zur Gestaltung und Implementation eines Softwarezustandsautomaten bekannt. Damit kann ein Zustandsautomateninitialisierungsprogramm geschaffen werden, das die Zustände, Bedingungen, Aktionen, auslösende Ereignisse und Zustandsübergänge für den Softwarezustandsautomaten definiert. Aus dem Zustandsautomaten wird ein Objekt erzeugt, das in andere Programme einbindbar ist. Ein solches Objekt veranlasst den Computer dann entsprechend dem Zustandsautomaten zu arbeiten.

US 2004/054297 A1 befasst sich mit einem neurologischen Implantat, das neurologische Ereignisse voraussagen soll. Ein Zustandsautomat, der konfigurierbar ist, wird für die Bestimmung der neurologischen Ereignisse benutzt.

Ausgehend von diesem Stand der Technik ist es Aufgabe der vorliegenden Erfindung, eine Versorgungseinrichtung bereitzustellen, die sich einfach programmieren lässt und eine Prüfung der Korrektheit der Programmierung schnell und effizient gewährleistet. Des Weiteren soll ein Verfahren zur Erzeugung eines Steuerprogramms für eine entsprechende Versorgungseinrichtung bereitgestellt werden.
Diese Aufgabe wird durch eine Versorgungseinrichtung nach Anspruch 1 gelöst. Insbesondere wird die Aufgabe durch eine Versorgungseinrichtung zum Betreiben mindestens eines medizinischen Instruments, insbesondere eines elektrochirurgischen Instruments und/oder eines kryochirurgischen Instruments und/oder eines wasserstrahlchirurgischen Instruments gelöst, wobei die Versorgungseinrichtung umfasst:
- eine Steuereinheit zur Steuerung des mindestens einen Instruments,
- eine Speichereinheit zum Speichern von Konfigurationsdaten, die einen Zustandsautomaten mit einer Vielzahl von Zuständen beschreiben,
wobei die Steuereinheit derart ausgebildet ist, dass sie die Konfigurationsdaten einliest, den Zustandsautomaten in ein Steuerprogramm übersetzt und das mindestens eine Instrument gemäß dem Steuerprogramm steuert.

Ein wesentlicher Gedanke der vorliegenden Erfindung besteht also darin, ein Rahmenprogramm oder Framework innerhalb der Versorgungseinrichtung vorzusehen, das das Einlesen und Umsetzen eines Steuerprogramms für die Instrumente in Form eines Zustandsautomaten ermöglicht. Dieses Rahmenprogramm muss üblicherweise nicht verändert werden, um die Funktionsweise der Versorgungseinrichtung weiter zu entwickeln. Die eigentliche Steuerung der Instrumente erfolgt anhand des Zustandsautomaten, der durch die Konfigurationsdaten beschrieben wird. Die Korrektheit eines Zustandsautomaten lässt sich sehr einfach validieren. Es werden vorzugsweise deterministische Automaten verwendet, so dass einfach überprüft werden kann, ob dieser Automat korrekt arbeitet. Aufgrund der Verwendung von Zustandsautomaten lässt sich der Arbeitsaufwand bei der Entwicklung von neuen Steuer- und Regelalgorithmen minimieren, wobei stets die Sicherheit des Patienten und des die Versorgungseinrichtung bedienenden Personals gewährleistet wird.

In einem Ausführungsbeispiel weist die Versorgungseinrichtung eine Schnittstelle zum Beschreiben der Speichereinrichtung mit den Konfigurationsdaten auf. Somit können bereits in Betrieb genommene Versorgungseinrichtungen auf einfache Weise mit aktuellen Regel- bzw. Steuerprogrammen versorgt werden. Es ist sehr einfach möglich, neue Betriebsmodi, beispielsweise einen Betriebsmodus zum Koagulieren von Lebergewebe, bereitzustellen. Mindestens einem Zustand des Zustandsautomaten kann mindestens ein Sollwert zugeordnet sein, wobei die Steuereinheit derart ausgebildet ist, dass sie beim Steuern des Instruments in einem Steuermodus gemäß dem Zustand mit dem mindestens einen Sollwert Steuersignale ausgibt, um das mindestens eine Instrument gemäß dem Sollwert zu steuern. Den Zuständen des Zustandsautomaten kann also ein oder mehrere Sollwert(e) durch die Konfigurationsdaten zugeordnet werden. Dieser Satz von Sollwerten kann die einzelnen Zustände definieren und von der Steuereinheit derart verarbeitet werden, dass die einzelnen Instrumente gemäß diesen Sollwerten betrieben bzw. gesteuert werden.

Die Versorgungseinrichtung kann eine Messeinrichtung zum Bestimmen mindestens eines Sensorsignals umfassen, das mindestens einem Sollwert zugeordnet ist, wobei die Steuereinheit das Instrument derart steuert, dass der Sollwert im Wesentlichen eingehalten wird. Gemäß dieser Anmeldung können Sensorsignale Aufschluss über von der Steuereinheit eingestellte Stellwerte, gemessene Parameter (z.B. ein Gewebewiderstand) und versorgungseinrichtungsinterne Signale (beispielsweise ein Zeitsignal) sein. Die den Zuständen zugeordneten Sollwerte werden also dazu verwendet, um bestimmte Stellgrößen vorzugeben und/oder Steuersignale auszugeben, so dass Messwerte ein bestimmtes vorgegebenes Kriterium erfüllen. Häufig lassen sich nicht alle Sollwerte erreichen, so dass die Steuereinheit dazu ausgebildet sein muss, um das oder die Instrumente nahe an einem optimalen Arbeitspunkt zu steuern. Ein optimaler Arbeitspunkt kann beispielsweise so definiert sein, dass die Summe der Beträge des absoluten Fehlers zwischen Sollwert und Istwert gemäß dem Sensorsignal möglichst gering ist. Die einzelnen Betragswerte der Fehler können auch gewichtet werden.

Die Steuereinheit kann zur Etablierung eines Arbeitspunkts ausgebildet sein, bei dem das jeweilige Sensorsignal den zugeordneten Sollwert nicht überschreitet.

Der Zustandsautomat kann mindestens eine Transition von mindestens einem ersten Zustand in mindestens einen zweiten Zustand umfassen, wobei die Konfigurationsdaten mindestens eine Transitionsregel enthalten und die Steuereinheit derart ausgebildet ist, dass sie aus einem ersten Steuermodus gemäß dem ersten Zustand in einen zweiten Steuermodus gemäß dem zweiten Zustand wechselt, wenn die Transitionsregel erfüllt ist. Durch das Vorhandensein der Transitionsregeln kann ein flexibler Wechsel zwischen den einzelnen Zuständen ermöglicht werden. Die Steuereinheit prüft die einzelnen Transitionsregeln ab und leitet einen Übergang von einem ersten Zustand in einen zweiten Zustand ein, wenn die Transitionsregel erfüllt ist. Für die Steuerung des Instruments bedeutet dies, dass aus einem ersten Steuermodus in einen zweiten Steuermodus gewechselt wird, wobei der jeweilige Steuermodus durch die den Zuständen zugeordneten Sollwerte definiert wird.

Dem ersten Zustand kann mindestens ein erster Sollwert und dem zweiten Zustand mindestens ein zweiter Sollwert zugeordnet sein, wobei sich der erste und der zweite Sollwert auf dasselbe Sensorsignal beziehen und die Steuereinheit derart ausgebildet ist, dass sie anhand der Konfigurationsdaten erfasst, wie eine Überführung des Sensorsignals vom ersten Sollwert zu dem zweiten Sollwert zu erfolgen hat und eine entsprechende Steuerung vornimmt. Üblicherweise wird bei dem Wechsel zwischen dem ersten und dem zweiten Steuermodus ein bestimmter Sollwert verändert. Erfindungsgemäß sollen die Konfigurationsdaten angeben, wie eine Überführung des Sollwerts vom ersten Wert zu dem zweiten Wert erfolgen soll, so dass entsprechende Steuersignale zur Steuerung des Instruments ausgesandt werden können. Beispielsweise kann der Sollwert innerhalb einer bestimmten Zeit mittels einer beispielsweise linearen Gleichung aufgerampt werden.
Die Transitionsregeln können mindestens eine Bedingung umfassen, die sich auf mindestens ein Sensorsignal bezieht. Ein Wechsel zwischen den Zuständen erfolgt also dann, wenn die Bedingungen erfüllt sind. Mindestens einem Zustand können mindestens zwei Transitionen zugeordnet sein. Den Transitionen kann jeweils eine Ordnung mittels der Konfigurationsdaten zugeordnet werden, wobei die Steuereinheit derart ausgebildet ist, dass bei einem Steuern des Instruments in einem Steuermodus gemäß dem Zustand mit den mindestens zwei zugeordneten Transitionen die Bedingungen der Transitionen gemäß der Ordnung abgeprüft werden. Das heißt, soweit ein Zustand mehrere Transitionen hat, die diesen in einen Folgezustand überführen können, ist es möglich, diese Transitionen mit einer Ordnung zu versehen. Die Ordnung informiert die Steuereinheit darüber, welche Transition zuerst abgeprüft werden muss und im Falle eines Erfüllens der zugeordneten Transitionsregel eingeleitet wird. Somit kann ein entsprechendes Steuerprogramm für die Instrumente flexibler gestaltet werden.

Die Konfigurationsdaten umfassen mindestens eine Steuertabelle. Der Zustandsautomat lässt sich mittels einer Steuertabelle relativ einfach modellieren.

Ein nicht erfindungsgemäßes Verfahren zur Erzeugung eines Steuerprogramms für eine Versorgungseinrichtung mindestens eines medizinischen Instruments wird beschrieben. Das Verfahren umfasst die Schritte:
- Einlesen von Konfigurationsdaten, die einen Zustandsautomaten mit einer Vielzahl von Zuständen beschreiben;
- Übersetzen des Zustandsautomaten mit der Vielzahl von Zuständen in ein Steuerprogramm durch eine Steuereinheit;
- Steuern des mindestens einen medizinischen Instruments gemäß dem Steuerprogramm durch die Steuereinheit.

Auch für dieses Verfahren ergeben sich ähnliche Vorteile, wie diese bereits in Verbindung mit der Versorgungseinrichtung beschrieben wurden.

Insbesondere lässt sich die Software für die Versorgungseinrichtung leichter pflegen und warten. Neue Regel- und Steuerungsprogramme können schnell und einfach modelliert werden. Es lassen sich einfache grafische Darstellungen erzeugen, die die Programme beschreiben. Somit lässt sich das Auftreten von nicht entdeckten logischen Fehlern reduzieren. Des Weiteren kann eine strenge Trennung zwischen dem beschriebenen Rahmenprogramm zur Implementierung des Zustandsautomaten und des eigentlichen Zustandsautomaten, der maßgebend für die Steuerung ist, hergestellt werden. Das dem Steuerprogramm zugrunde liegende Rahmenprogramm wird in den meisten Fällen nicht verändert, es fungiert als gekapselte Blackbox. Nach einer erfolgreichen Validierung dieses Rahmenprogramms gestalten sich Aktualisierungen des Systems sehr einfach.

Das Verfahren kann ein Beschreiben eines internen Speichers der Versorgungseinrichtung mit Konfigurationsdaten eines externen Speichers umfassen. Somit lässt sich die beschriebene Aktualisierung des Steuerprogramms leicht durchführen.

Das Verfahren kann beim Steuern des mindestens einen Instruments gemäß einem aktuellen Zustand aus der Vielzahl von Zuständen folgende Schritte umfassen:
- Bestimmen eines Sensorsignals mittels einer Messeinrichtung;
- Vergleichen des Sensorsignals mit einem dem aktuellen Zustand zugeordneten Sollwert;
- Ausgeben eines Steuersignals, um das Sensorsignal gemäß dem Sollwert einzustellen.

Somit lassen sich die Zustände der Zustandsmaschine beispielsweise durch Sollwerte definieren.

Weitere vorteilhafte Ausführungsformen ergeben sich anhand der Unteransprüche.

Nachfolgend wird die Erfindung mittels mehrerer Ausführungsbeispiele beschrieben, die anhand von Abbildungen näher erläutert werden. Hierbei zeigen:
Fig. 1 eine Versorgungseinrichtung mit angeschlossenem elektrochirurgischem Instrument;
Fig. 2 einzelne Komponenten der Versorgungseinrichtung; und
Fig. 3 einen Zustandsautomaten zur Implementierung durch die Versorgungseinrichtung.

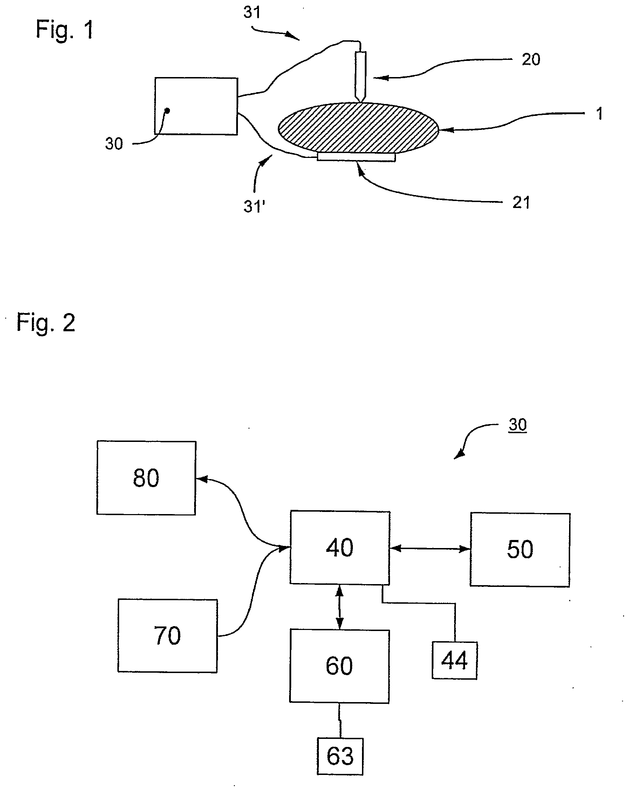

In der nachfolgenden Beschreibung werden für gleiche und gleich wirkende Teile dieselben Bezugsziffern verwendet. Die Fig. 1 zeigt eine Versorgungseinrichtung 30, die über eine erste HF-Leitung 31 mit einem elektrochirurgischen Instrument 20 verbunden ist. Eine zweite HF-Leitung 31' führt zu einer Neutralelektrode 21. Zum Anlegen einer HF-Spannung U, die von der Versorgungseinrichtung 30 bereitgestellt wird, umfasst das Instrument 20 eine weitere Elektrode. Somit kann die HF-Spannung U zwischen der Neutralelektrode 21 und der weiteren Elektrode angelegt werden. Wie in der Fig. 1 gezeigt, können die Neutralelektrode 21 und die weitere Elektrode des elektrochirurgischen Instruments 20 verwendet werden, um einen HF-Strom I in ein biologisches Gewebe einzubringen. Die Fig. 1 zeigt einen Torso 1, an dem die selbstklebende Neutralelektrode 21 angebracht ist. Die weitere Elektrode des elektrochirurgischen Instruments 20 wird dazu verwendet, das Gewebe auf der anderen Seite des Torso 1 zu schneiden oder zu koagulieren. Wie in Fig. 2 gezeigt, umfasst die Versorgungseinrichtung 30 eine Steuereinheit 40 und einen HF-Generator 50. Der HF-Generator 50 dient dazu, eine geeignete HF- Spannung U für das elektrochirurgische Instrument 20 und die Neutralelektrode 21 bereitzustellen. Die Steuereinheit 40 steuert den HF-Generator 50, indem sie Steuersignale an diesen ausgibt und empfängt von diesem Sensorsignale, die Auskünfte über den Zustand des HF-Generators 50 sowie über den applizierten HF-Strom I und die HF-Spannung U geben. Die empfangenen Sensorsignale können auch Aufschluss über eine Gewebeimpedanz R, eine HF-Leistung P oder einen Wirkleistungsfaktor cos [phi] geben. Die Steuereinheit 40 ist vorzugsweise dazu ausgebildet, mehrere unterschiedliche Betriebsmodi bereitzustellen, die sich beispielsweise auf die zu applizierende HF-Spannung U oder HF-Leistung P auswirken.

So kann die Steuereinheit 40 einen Modus zum Koagulieren und einen weiteren Modus zum Schneiden von Gewebe anbieten. Des Weiteren können weitere Modi für unterschiedliche elektrochirurgische Instrumente 20 (beispielsweise für monopolare oder bipolare Instrumente) oder für unterschiedliche Gewebetypen (Leber oder Muskelgewebe) bereitgestellt werden. Die Versorgungseinrichtung 30 umfasst eine Eingabeeinheit 70, die es ermöglicht, Eingaben von einem Benutzer des elektrochirurgischen Instruments 20 zu empfangen. Der Benutzer kann also über die Eingabeeinheit 70 einen bestimmten Betriebsmodus auswählen und die Steuereinheit 40 derart aktivieren, dass der HF-Generator 50 und somit das elektrochirurgische Instrument 20 in diesem Betriebsmodus betrieben wird. Um die Auswahl eines bestimmten Betriebsmodus zu erleichtern und die Statusinformationen bezüglich des HF-Generators 50 und/oder der Steuereinheit 40 und/oder des elektrochirurgischen Instruments 20 anzuzeigen, umfasst die Versorgungseinrichtung 30 des Weiteren eine Ausgabeeinheit 80. Die Ausgabeeinheit 80 enthält einen Bildschirm zum Anzeigen von Information.

Die Steuereinheit 40 steht des Weiteren in kommunikativer Verbindung mit einer Speichereinheit 60, die über eine Schnittstelle 63 verfügt, durch die Konfigurationsdaten in die Speichereinheit 60 geladen werden können. Die Steuereinheit 40 hat eine Zeiterfassungseinheit 44, die Sensorsignale in Form von Zeitsignalen vorgibt, die das Erfassen von vorgegebenen Zeitintervallen ermöglichen.

Erfindungsgemäß enthält die Speichereinheit 60 ein Rahmenprogramm, das durch die Steuereinheit 40 ausgeführt wird. Dieses Rahmenprogramm veranlasst die Steuereinheit 40, Konfigurationsdaten aus der Speichereinheit 60 zu laden und ein Steuerprogramm zu generieren, das die Steuerung des an die Versorgungseinrichtung 30 angeschlossenen Instruments 20 ermöglicht. Vorzugsweise wird hierbei ein tabellengesteuertes Schaltwerk implementiert, das eine Vielzahl von Zuständen Z0 bis Z4 hat, wobei die einzelnen Zustände Z0 bis Z4 in Abhängigkeit von vorgegebenen Randbedingungen eingenommen werden.

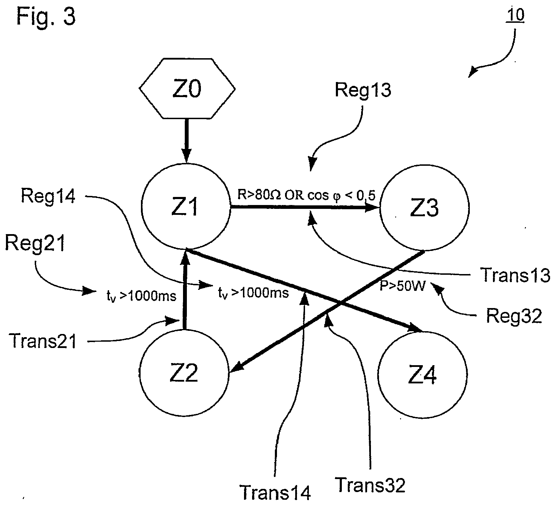

Die in der Speichereinheit 60 gespeicherten Konfigurationsdaten können hierbei tabellarisch einen Zustandsautomaten 10 modellieren. Ein entsprechender Zustandsautomat 10 ist in der Fig. 3 gezeigt. Er umfasst die Zustände Z0 bis Z4, wobei der Startzustand Z0 beispielsweise nach der Auswahl eines bestimmten Koagulationsmodus eingenommen wird. Ausgehend von dem Startzustand Z0 geht der Zustandsautomat in einen ersten Zustand ZI über. Der Zustandsautomat 10 hat eine Vielzahl von Transitionen Trans13, Trans14, Trans21, Trans32, die einen Übergang in weitere Zustände, nämlich einen zweiten Zustand Z2, einen dritten Zustand Z3, einen vierten Zustand Z4 modellieren. So beschreibt die Transition Transl3 beispielsweise einen Übergang aus dem ersten Zustand ZI in den dritten Zustand Z3. Jede dieser Transitionen Transl3, Transl4, Trans21, Trans32 ist einer Transitionsregel Reg13 bzw. Reg14 bzw. Reg21 bzw. Reg32 zugeordnet. Die Transitionsregel Regl3 gehört zu der Transition Transl3 und enthält zwei Bedingungen, bei deren Erfüllung die Steuereinheit 40 aus einem Steuermodus gemäß dem ersten Zustand ZI in einen Steuermodus gemäß dem dritten Zustand Z3 wechselt. Im in der Fig. 3 beschriebenen Fall wird die Transition Transl3 vorgenommen, wenn die Impedanz R größer als 80 Ohm ist oder der Wirkleistungsfaktor cos [phi] größer als 0,5. Es sind Transitionsregeln Reg13, Reg14, Reg21, Reg32 denkbar, bei denen eine Bedingung über einen vorgegebenen Zeitraum eingehalten werden muss. Beispielsweise ist es denkbar, dass die Transition Transl3 nur dann vorgenommen wird, wenn der Wirkleistungsfaktor cos [phi] für mehr als 5 Millisekunden größer als 0,5 ist. Ausgehend von dem ersten Zustand ZI sind weitere Transitionen Trans13, Trans14, Trans21, Trans32 möglich. Eine weitere Transition Transl4 führt aus dem ersten Zustand in den vierten Zustand. Der Transition Trans14 ist die Transitionsregel Reg14 zugeordnet. Die Transition Trans14 findet nach Transitionsregel Reg14 statt, wenn die Steuereinheit 40 in dem Steuermodus gemäß dem ersten Zustand ZI länger als 1000 Millisekunden verblieben ist (Verweildauer tv ist größer als 1000 Millisekunden). Die Transition Trans32 führt von dem dritten Zustand Z3 zu dem zweiten Zustand Z2 und wird gemäß der Transitionsregel Reg32 dann vorgenommen, wenn eine HF-Leistung P grösser als 50 Watt ist. Die Transition Trans21 führt von dem zweiten Zustand Z2 in den dritten Zustand Z3 und findet nach einer Einnahme des zweiten Zustands Z2 für einen Zeitraum länger als 1000 Millisekunden statt (Bedingung: Verweildauer tv größer 1000 Millisekunden). Diese Bedingung wird durch die Transitionsregel Reg21 angegeben. Insgesamt modelliert der Zustandsautomat 10 einen abgeschlossenen Regel- bzw. Steueralgorithmus, der die Steuereinheit 40 aus einem Startzustand Z0 über mehrere Zwischenzustände ZI, Z2, Z3 in einen Endzustand, nämlich den vierten Zustand Z4 führt.

Jedem der Zustände ZI bis Z4 sind vorzugsweise in tabellarischer Form Sollwerte zugeordnet:
Sollwertsatz für Zustand ZI:
   U = 200 V
   I = 3 A
   P = 120 W
Sollwertsatz für Zustand Z2:
   U = 350 V
   I = 1 A
   P = 300 W
Sollwertsatz für Zustand Z3: U = 500 V
   I = 1 A
   P = 120 W
Sollwertsatz für Zustand Z4:
   U = 250 V
   I = 3 A
   P = 90 W

Die Steuereinheit 40 kann so konfiguriert sein, dass die einzelnen Sollwerte als Stellwerte eingestellt werden. Beispielsweise kann die Steuereinheit 40 Steuersignale an den HF-Generator 50 abgeben, die diesen so einstellen, dass die Sollwerte erfüllt werden. Häufig ist ein Betreiben der Versorgungseinrichtung 30 bei den gegebenen Sollwerten jedoch nicht möglich. Für diese Fälle ist die Steuereinheit 40 derart konfiguriert, dass sie Arbeitspunkte etabliert, bei denen Einstellungen oder Messwerte erzielt werden, die möglichst nahe an den vorgegebenen Sollwerten liegen.

In dem vorab genannten Ausführungsbeispiel gehen von dem ersten Zustand ZI mehrere Transitionen, nämlich die Transition Transl3 und Trans14 aus. Es ist möglich, den einzelnen Transitionen Trans13, Transl4 Prioritäten zuzuordnen, so dass diese nach einer gegebenen Ordnung von der Steuereinheit 40 überprüft werden. Beispielsweise kann die Transition Transl3 vor der Transition Trans14 überprüft werden. Sollten also die Bedingungen gemäß der Transitionsregel Regl3 zu einem gegebenen Zeitpunkt, bei dem sich die Steuereinheit 40 in einem Steuermodus gemäß dem ersten Zustand ZI befindet erfüllt sein, so wird die Steuereinheit sofort in einen Steuermodus gemäß dem dritten Zustand Z3 übergehen. Nur wenn die Bedingungen der Transitionsregel Regl3 nicht erfüllt sind, wird die Transitionsregel Reg 14 überprüft.

Allgemein kann ein erfindungsgemäßer Steuerungsautomat bzw. Zustandsautomat über eine Steuertabelle parametrisiert werden, die umfasst:

### 1. Starteinstellung

Beispielsweise mit einem Sollwert für den Startzustand Z0.

### 2. Weitere Zustände als Folgezustände

Beispielsweise die Zustände ZI bis Z4.

### 3. Übergänge:

Beispielsweise die Transitionen Transl3, Transl4, Trans21, Trans32.

### 4. Übergangsbedingungen

Beispielsweise die Transitionsregeln Reg13, Reg14, Reg21, Reg32.

Die Übergangsbedingungen können sich auf Schwellwerte oder Änderungssteilheiten von Messgrößen oder auf absolute Werte (z.B. R größer 200 Ohm) oder relative Werte (z.B. R größer 10 % des letzten gemessenen Werts) beziehen. Alternativ können Häufigkeiten (z.B. bei wie vielen Messungen war R größer 200 Ohm) oder Zeitintervalle (Verweildauer tv = 300 Millisekunden) berücksichtigt werden. Die Übergangsbedingungen können auch Kombinationen von Übergangsbedingungen (beispielsweise logische UND/ODER-Verknüpfungen) beinhalten.

Nachfolgend wird eine beispielhafte Steuertabelle für das Instrument 20 dargestellt, die im Wesentlichen der Konfiguration nach Fig. 3 entspricht:
*** Grundeinstellung: ***
U_HF max [Vpeak] : = 200
I_HF max [A rms] : = 3
P_HF max [W] : = 120
RiGenerator[Ohm] : = 0
Funke [V] : = 0
Signale, Relais := Cut/Coag CW
U_Nt max [V] : = 200
I_Nt max [A] : = 2,5
APC Argon[l/min] : = 0
APC Preflow [s] : = 0
Zeitraster der Sprungbedingungen = lOOus
Zustand 1 : Start
-> U_HF max : - 200Vpeak
-> I_HF max : = 3A rms
-> P_HF max : = 120W
Sprung 1A> >> Widerstand überschritten < < < Sprung 1A: Wenn 1 mal RLast >= 80 Ohm, dann wechsle zu Zustand: 3 Sprung 1B>> > cos phi < 0,5 (=LF 16384) << <
Sprung 1B: Wenn 50 mal LF < 16384 , dann wechsle zu Zustand: 3 Sprung 1C> > > Zeitlimit < < <
Sprung IC: Wenn 10000 mal, dann wechsle zu Zustand: 4
Zustand 2 : Zustand 2
-> U_HF max : = 350Vpeak
-> I_HF max : = 1A rms
-> P_HF max : = 300W
Sprung 2A> > > 1 Sekunde warten < < <
Sprung 2A: Wenn 10000 mal, dann wechsle zu Zustand: 3
Zustand 3 : Zustand 3
-> U_HF max : = 500Vpeak
-> I_HF max := 1A rms
-> P_HF max : = 120W
Sprung 3A> > > Leistung überschritten < < <
Sprung 3A: Wenn 1 mal P_HF > 50W, dann wechsle zu Zustand: 2
Zustand 4 : Zustand 4
> >> Stabiler Zustand bis Ende Aktivierung < < <
-> U_HF max : = 250Vpeak
-> I_HF max : = 3A rms
-> P_HF max : = 90W

In den vorhergehenden Ausführungsbeispielen wurden zur Veranschaulichung der Erfindung Steuerprogramme für elektrochirurgische Instrumente beschrieben. Es sollte jedoch für den hier tätigen Fachmann ohne weiteres klar sein, wie sich die Erfindung zur Steuerung von kryochirurgischen Instrumenten und/oder wasserstrahlchirurgischen Instrumenten verwenden lässt.

In diesem Zusammenhang muss auch festgestellt werden, dass sich die Sollwerte auch auf mechanische Tätigkeiten, beispielsweise das Aktivieren und Deaktivieren eines Ventils, beziehen können. So kann beispielsweise ein Sollwert angeben, dass ein Ventil geöffnet oder geschlossen wird. Des Weiteren ist es denkbar, dass ein Sollwert eine Position in einem dreidimensionalen Koordinatensystem angibt, die von einem Gerät, beispielsweise einem Roboterarm, angefahren werden soll. Auch kann der Sollwert angeben, dass die Spitze eines elektrochirurgischen Instruments aus- oder eingefahren wird. Somit können die einzelnen Zustände mit einer Vielzahl von mechanischen Tätigkeiten, die von dem elektrochirurgischen Instrument oder der Versorgungseinrichtung vorgenommen werden, verknüpft werden.

### Bezugszeichenliste

1 Torso
10 Zustandsautomat
20 elektrochirurgisches Instrument
21 Neutralelektrode
30 Versorgungseinrichtung
31, 31' HF-Leitung
40 Steuereinheit
44 Zeiterfassungseinheit
50 HF-Generator
60 Speichereinheit
63 Schnittstelle
70 Eingabeeinheit
80 Ausgabeeinheit
ZO Startzustand
ZI erster Zustand
Z2 zweiter Zustand
Z3 dritter Zustand
Z4 vierter Zustand
Regl3 Transitionsregel von ZI nach Z3
Reg14 Transitionsregel von ZI nach Z4
Reg21 Transitionsregel von Z2 nach ZI
Reg32 Transitionsregel von Z3 nach Z2
Trans13, Trans14,
Trans 21, Trans32 Transition
U HF-Spannung
I HF-Strom
P HF-Leistung
R Impedanz Wirkleistungsfaktor Verweildauer

## Patentansprüche

1. Versorgungseinrichtung zum Betreiben mindestens eines elektrochirurgischen Instruments (20) und/oder eines kryochirurgischen Instruments und/oder wasserstrahlchirurgischen Instruments, umfassend:
- eine Steuereinheit (40) zur Steuerung des mindestens einen Instruments (20),
- eine Speichereinheit (60) zum Speichern von Konfigurationsdaten, die einen Zustandsautomaten (10) mit einer Vielzahl von Zuständen (Z0-Z4) beschreiben, **dadurch gekennzeichnet, dass** die Steuereinheit (40) derart ausgebildet ist, dass sie die Konfigurationsdaten einliest, die mindestens eine Steuertabelle umfassen, den Zustandsautomaten in ein Steuerprogramm übersetzt und das mindestens eine Instrument (20) gemäß dem Steuerprogramm steuert.

2. Versorgungseinrichtung nach Anspruch 1, **gekennzeichnet durch** eine Schnittstelle (63) zum Beschreiben der Speichereinrichtung (60) mit den Konfigurationsdaten.

3. Versorgungseinrichtung nach einem der vorhergehenden Ansprüche, **dadurch gekennzeichnet, dass** mindestens einem Zustand (Z0-Z4) des Zustandsautomaten (10) mindestens ein Sollwert zugeordnet ist und die Steuereinheit (40) derart ausgebildet ist, dass sie beim Steuern des Instruments (20) in einem Steuermodus gemäß dem Zustand (Z0-Z4) mit dem mindestens einen Sollwert Steuersignale ausgibt, um das mindestens eine Instrument (20) gemäß dem Sollwert zu steuern.

4. Versorgungseinrichtung nach einem der vorhergehenden Ansprüche, insbesondere nach Anspruch 3, **gekennzeichnet durch** eine Messeinrichtung zum Bestimmen mindestens eines Sensorsignals, das mindestens einem Sollwert zugeordnet ist, wobei die Steuereinheit (40) das Instrument (10) derart steuert, dass der Sollwert im Wesentlichen eingehalten wird.

5. Versorgungseinrichtung nach einem der vorhergehenden Ansprüche, insbesondere nach Anspruch 4, **dadurch gekennzeichnet, dass** die Steuereinheit zur Etablierung eines Arbeitspunktes ausgebildet ist, bei dem das jeweilige Sensorsignal den zugeordneten Sollwert nicht überschreitet.

6. Versorgungseinrichtung nach einem der vorhergehenden Ansprüche, **dadurch gekennzeichnet, dass** der Zustandsautomat (10) mindestens eine Transition (Trans13, Trans14, Trans21, Trans32) von mindestens einem ersten Zustand (Z0-Z4) in mindestens einen zweiten Zustand (Z0-Z4) umfasst, wobei die Konfigurationsdaten mindestens eine Transitionsregel (Reg13, Reg14, Reg21, Reg32) umfassen, und die Steuereinheit (40) derart ausgebildet ist, dass sie aus einem ersten Steuermodus gemäß dem ersten Zustand (Z0-Z4) in einen zweiten Steuermodus gemäß dem zweiten Zustand (Z0-Z4) wechselt, wenn die Transitionsregel (Reg13, Reg14, Reg21, Reg32) erfüllt ist.

7. Versorgungseinrichtung nach einem der vorhergehenden Ansprüche, insbesondere nach Anspruch 6, **dadurch gekennzeichnet, dass** dem ersten Zustand (Z0-Z4) mindestens ein erster Sollwert und dem zweiten Zustand (Z0-Z4) mindestens ein zweiter Sollwert zugeordnet ist, wobei sich der erste und der zweite Sollwert auf dasselbe Sensorsignal bezieht und die Steuereinheit derart ausgebildet ist, dass sie anhand der Konfigurationsdaten erfasst, wie eine Überführung des Sensorsignals vom ersten Sollwert zu dem zweiten Sollwert zu erfolgen hat und eine entsprechende Steuerung vornimmt.

8. Versorgungseinrichtung nach einem der vorhergehenden Ansprüche, insbesondere nach Anspruch 6 oder 7, **dadurch gekennzeichnet, dass** mindestens eine Transitionsregel (Reg13, Reg14, Reg21, Reg32) mindestens eine Bedingung umfasst, die sich auf mindestens ein Sensorsignal bezieht.

9. Versorgungseinrichtung nach einem der vorhergehenden Ansprüche, **dadurch gekennzeichnet, dass**
mindestens einem Zustand (Z0-Z4) mindestens zwei Transitionen (Trans13, Trans14, Trans21, Trans32) zugeordnet sind und den Transitionen (Trans13, Trans14, Trans21, Trans32) jeweils eine Ordnung mittels der Konfigurationsdaten zugeordnet ist, wobei die Steuereinheit (40) derart ausgebildet ist, dass bei einem Steuern des Instruments (20) in einem Steuermodus gemäß dem Zustand mit den mindestens zwei zugeordneten Transitionen (Trans13, Trans14, Trans21, Trans32) die Bedingungen der Transitionen (Trans13, Trans14, Trans21, Trans32) gemäß der Ordnung abprüft.

## Claims

1. Supply device for operating at least one first electrosurgical instrument (20) and/or a cryosurgical instrument and/or a water jet surgical instrument, comprising:
- a control unit (40) for controlling the at least one instrument (20),
- a memory unit (60) for storing configuration data that describe a state machine (10) having a plurality of states (Z0-Z4),
**characterised in that**
the control unit (40) is configured such that it reads the configuration data comprising at least one control table, translates the state machine into a control program and controls the at least one instrument (20) according to the control program.

2. Supply device according to claim 1, **characterised by** an interface (63) for writing the configuration data to the memory device (60).

3. Supply device according to any of the preceding claims, **characterised in that** at least one nominal value is assigned to at least one state (Z0-Z4) of the state machine (10), and the control unit (40) is configured such that when controlling the instrument (20) in a control mode according to the state (Z0-Z4) with the at least one nominal value, it outputs control signals for controlling the at least one instrument (20) according to the nominal value.

4. Supply device according to any of the preceding claims, in particular claim 3, **characterised by** a measurement device for determining at least one sensor signal assigned to at least one nominal value, wherein the control unit (40) controls the instrument (10) such that the nominal value is substantially observed.

5. Supply device according to any of the preceding claims, in particular claim 4, **characterised in that** the control unit is configured to establish a working point at which the respective sensor signal does not exceed the assigned nominal value.

6. Supply device according to any of the preceding claims, **characterised in that** the state machine (10) comprises at least one transition (Transl3, Transl4, Trans21, Trans32) from at least one first state (Z0-Z4) into at least one second state (Z0-Z4), wherein the configuration data comprise at least one transition rule (Regl3, Regl4, Reg21, Reg32), and the control unit (40) is configured such that it changes from a first control mode according to the first state (Z0-Z4) into a second control mode according to the second state (Z0-Z4) when the transition rule (Reg13, Regl4, Reg21, Reg32) is fulfilled.

7. Supply device according to any of the preceding claims, in particular claim 6, **characterised in that** at least one first nominal value is assigned to the first state (Z0-Z4), and at least one second nominal value is assigned to the second state (Z0-Z4), wherein the first and the second nominal values concern the same sensor signal, and the control unit is configured such that it detects from the configuration data whether the sensor signal must be transferred from the first nominal value to the second nominal value, and performs a corresponding control.

8. Supply device according to any of the preceding claims, in particular claim 6 or 7, **characterised in that** at least one transition rule (Reg13, Regl4, Reg21, Reg32) comprises at least one condition which concerns at least one sensor signal.

9. Supply device according to any of the preceding claims, **characterised in that** at least two transitions (Trans 13, Trans 14, Trans21, Trans32) are assigned to a state (Z0-Z4), and by means of configuration data a respective order is assigned to the transitions (Trans13, Trans14, Trans21, Trans32), wherein the control unit (40) is configured such that when controlling the instrument (20) in a control mode according to the state with the at least two assigned transitions (Trans13, Trans14, Trans21, Trans32), it checks the conditions of the transitions (Trans 13, Trans 14, Trans21, Trans32) according to the order.

## Revendications

1. Dispositif d'alimentation pour faire fonctionner au moins un instrument électrochirurgical (20) et/ou un instrument cryochirurgical et/ou un instrument chirurgical à jet d'eau, comprenant :
- une unité de commande (40) destinée à contrôler l'instrument (20), au nombre d'au moins un,
- une unité de mémoire (60) destinée à stocker des données de configuration qui décrivent un automate d'état (10) avec une pluralité d'états (Z0-Z4),
**caractérisé en ce que** l'unité de commande (40) est conçue pour entrer les données de configuration qui comprennent au moins une table de commande, pour faire passer l'automate d'état à un programme de commande et pour contrôler l'instrument (20), au nombre d'au moins un, conformément au programme de commande.

2. Dispositif d'alimentation selon la revendication 1, **caractérisé par** une interface (63) destinée à inscrire les données de configuration dans le dispositif de mémoire (60).

3. Dispositif d'alimentation selon l'une des revendications précédentes, **caractérisé en ce qu'**au moins une valeur de consigne est associée à au moins un état (Z0-Z4) de l'automate d'état (10), et que l'unité de commande (40) est conçue de manière telle que lorsque l'instrument (20) est commandé dans un mode de commande conforme à l'état (Z0-Z4) avec la valeur de consigne, au nombre d'au moins une, elle émette des signaux de commande aux fins de commander l'instrument (20), au nombre d'au moins un, conformément à la valeur de consigne.

4. Dispositif d'alimentation selon l'une des revendications précédentes, notamment selon la revendication 3, **caractérisé par** un dispositif de mesure destiné à déterminer au moins un signal de capteur qui est associé à au moins une valeur de consigne, l'unité de commande (40) commandant l'instrument (10) de manière à ce que la valeur de consigne soit sensiblement respectée.

5. Dispositif d'alimentation selon l'une des revendications précédentes, notamment selon la revendication 4, **caractérisé en ce que** l'unité de commande est conçue pour établir un point de fonctionnement sur lequel le signal de capteur respectif ne dépasse pas la valeur de consigne associée.

6. Dispositif d'alimentation selon l'une des revendications précédentes, **caractérisé en ce que** l'automate d'état (10) comprend au moins une transition (Trans13, Trans14, Trans21, Trans32) d'au moins un premier état (Z0-Z4) à au moins un deuxième état (Z0-Z4), les données de configuration comprenant au moins une règle de transition (Reg13, Reg14, Reg21, Reg32), et l'unité de commande (40) étant conçue pour passer d'un premier mode de commande selon le premier état (Z0-Z4) à un deuxième mode de commande selon le deuxième état (Z0-Z4), lorsque la règle de transition (Reg13, Reg14, Reg21, Reg32) est remplie.

7. Dispositif d'alimentation selon l'une des revendications précédentes, notamment selon la revendication 6, **caractérisé en ce qu'**au moins une première valeur de consigne est associée au premier état (Z0-Z4) et au moins une deuxième valeur de consigne est associée au deuxième état (Z0-Z4), sachant que la première et la deuxième valeur de consigne se réfèrent au même signal de capteur et que l'unité de commande est conçue pour déterminer, à l'aide des données de configuration, de quelle manière doit s'effectuer la transition du signal de capteur de la première valeur de consigne à la deuxième valeur de consigne, et pour procéder à une commande correspondante.

8. Dispositif d'alimentation selon l'une des revendications précédentes, notamment selon la revendication 6 ou 7, **caractérisé en ce qu'**au moins une règle de transition (Reg13, Reg14, Reg21, Reg32) comprend au moins une condition qui se rapporte à au moins un signal de capteur.

9. Dispositif d'alimentation selon l'une des revendications précédentes, **caractérisé en ce qu'**au moins deux transitions (Trans13, Trans14, Trans21, Trans32) sont associées à au moins un état (Z0-Z4) et qu'un ordre est respectivement associé aux transitions (Trans13, Trans14, Trans21, Trans32) au moyen des données de configuration, l'unité de commande (40) étant conçue de manière à vérifier les conditions des transitions (Trans13, Trans14, Trans21, Trans32) conformément à l'ordre, lorsque l'instrument (20) est commandé dans un mode de commande conforme à l'état avec les au moins deux transitions (Trans13, Trans14, Trans21, Trans32).
